# EUROPEAN PATENT SPECIFICATION

(11) **EP 1 239 878 B1**
(45) Date of publication and mention of the grant of the patent: **25.02.2004**
(21) Application number: 00985623.8
(22) Date of filing: 19.12.2000
(51) Int. Cl.: A61K 45/06, A61P 25/00, A61P 29/00

(54) **FORMULATIONS OF ADENOSINE A1 AGONISTS AND 5HT1 AGONISTS**
ZUSAMMENSETZUNGEN VON ADENOSIN A1 AGONISTEN MIT 5HT1 AGONISTEN
FORMULATIONS D'AGONISTES DE L'ADENOSINE A1 ET D'AGONISTES DE 5HT1

(30) Priority: 20.12.1999 GB 9930085
(43) Date of publication of application: 18.09.2002
(73) Proprietor: GLAXO GROUP LIMITED, Greenford, Middlesex UB6 ONN (GB)
(72) Inventor: BOUNTRA, Charanjit, Stevenage Hertfordshire SG1 2NY (GB); CLAYTON, Nicholas Maughan, Stevenage Hertfordshire SG1 2NY (GB); NAYLOR, Alan, Stevenage Hertfordshire SG1 2NY (GB)
(74) Representative: Giddings, Peter John, Dr.
(86) International application number: PCT/GB2000/004878
(87) International publication number: WO 2001/045682

(56) References cited:
- WO-A-98/55453
- WO-A-99/66918

## Description

The present invention relates to the use of an adenosine A1 agonist in conjunction with a 5HT₁ receptor agonist in the manufacture of a medicament for the treatment of conditions associated with pain including acute pain, chronic pain, inflammatory pain, neuropathic pain and pain associated with migraine, tension headaches, cluster headaches and functional bowel disorder.

A variety of compounds which are agonists at the adenosine A1 receptor have been described in the art. These include compounds described in published patent applications WO99/24449, WO99/24450, WO99/24451, WO97/43300, WO98/16539, WO98/04126, WO98/01459, EP0322242, GB2226027, EP222330, WO98/08855, WO94/0707 and WO99/67262. WO99/67262 discloses compounds of formula (I) which are agonists at the adenosine A1 receptor 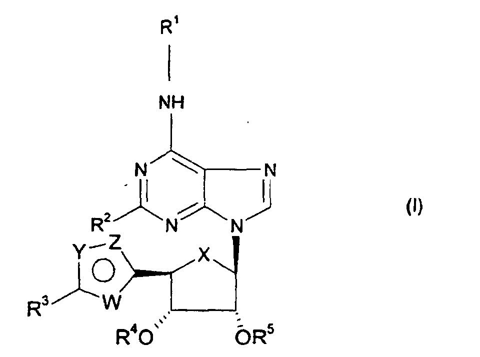 wherein X represents O or CH₂;
R² represents C₁₋₃alkyl, C₁₋₃alkoxy, halogen or hydrogen;
R³ represents H, phenyl (optionally substituted by halogen), a 5 or 6 membered heteroaryl group, C₁₋₆ alkoxy, C₁₋₆ alkylO(CH₂)ₙ where n is 0-6, C₃₋₇ cycloalkyl, C₁₋₆ hydroxyalkyl, halogen or a C₁₋₆ straight or branched alkyl, C₁₋₆ alkenyl or C₁₋₆ alkynyl group optionally substituted by one or more halogens.

Y and Z represent O, N, CH, N(C₁₋₆ alkyl)
W represents CH, O, N, S, N(C₁₋₆ alkyl)
and wherein at least one of W and Z represents a heteroatom (and when Y, Z and/or W is N, the presence or absence of an additional H would be apparent to a person skilled in the art)
with the proviso that when W represents CH, Z represents N and Y represents O, R³ cannot be H.

R⁴ and R⁵ independently represent H or a C₁₋₆ straight chain or branched alkyl group.

R¹ represents hydrogen or a group selected from
(1) -(alk)ₙ - (C₃₋₇) cycloalkyl, including bridged cycloalkyl, said cycloalkyl group optionally substituted by one or more substituents selected from OH, halogen, -(C₁₋₃) alkoxy, wherein (alk) represents C₁₋₃ alkylene and n represents 0 or 1.
(2) an aliphatic heterocyclic group of 4 to 6 membered rings containing at least one heteroatom selected from O, N or S, optionally substituted by one or more substituents selected from the group consisting of -(C₁₋₃)alkyl, -CO₂-(C₁₋₄)alkyl, -CO(C₁₋₃alkyl), -S(=O)ₙ-(C₁₋₃alkyl), -CONR^{a}R^{b} (wherein R^{a} and R^{b} independently represent H or C₁₋₃alkyl) or =O; where there is a sulfur atom in the heterocyclic ring, said sulfur is optionally substituted by (=O)ₙ, where n is 1 or 2.
(3) Straight or branched C₁₋₁₂ alkyl, optionally including one or more O, S(=O)ₙ (where n is 0, 1 or 2) and N groups substituted within the alkyl chain, said alkyl optionally substituted by one or more of the following groups, phenyl, halogen, hydroxy, C₃₋₇ cycloalkyl or NR^{a}R^{b} wherein R^{a} and R^{b} independently represent hydrogen, C₃₋₇ cycloalkyl or a C₁₋₆ straight chain or branched alkyl optionally substituted by C₃₋₇ cycloalkyl;
(4) a fused bicyclic aromatic ring 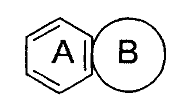 wherein B represents a 5 or 6 membered heterocyclic aromatic group containing 1 or more O, N or S atoms, wherein the bicyclic ring is attached to the nitrogen atom of formula (I) via a ring atom of ring A and ring B is optionally substituted by -CO₂ -(C₁₋₃alkyl).
(5) a phenyl group optionally substituted by one or more substituents selected from:
   - halogen, -SO₃H, -(alk)ₙOH, -(alk)ₙ -cyano, -(O)ₙ -(C₁₋₆)alkyl (optionally substituted by one or more halogens), - (alk)ₙ -nitro, -(O)ₘ -(alk)ₙ- CO₂R^{c}, -(alkₙ)- CONR^{c}R^{d} -(alk)ₙ -COR^{c}, -(alk)ₙ -SOR^{e}, -(alk)ₙ -SO₂R^{e}, -(alk)ₙ-SO₂NR^{c}R^{d}, -(alk)ₙOR^{c}, -(alk)ₙ -(CO)ₘ- NHSO₂R^{e}, -(alk)ₙ- NHCOR^{c}, -(alk)ₙ-NR^{c}R^{d} wherein m and n are 0 or 1 and alk represents a C₁₋₆alkylene group or C₂₋₆ alkenyl group.
(6) A phenyl group substituted by a 5 or 6 membered heterocyclic aromatic group, said heterocyclic aromatic group optionally being substituted by C_{1- 3}alkyl or NR^{c}R^{d}.

R^{c} and R^{d} may each independently represent hydrogen, or C₁₋₃ alkyl or when part of a group NR^{c}R^{d}, R^{c} and R^{d} together with the nitrogen atom may form a 5 or 6 membered heterocyclic ring optionally containing other heteroatoms, which heterocyclic ring may optionally be substituted further by one or more C₁₋₃ alkyl groups.

R^{e} represents C₁₋₃alkyl
and salts and solvates thereof, in particular, physiologically acceptable solvates and salts thereof for use in therapy.

These compounds are described as being useful in the treatment of pain, cardiac disorders, CV disorders including hyperlipidemia, diabetes, sleep apnoea, CNS disorders including epilepsy. The above applications also describe in relation to the adenosine A1 agonists they disclose both suitable methods for their preparation and doses for their administration.

We have now found that administration of an adenosine A1 agonist in conjunction with 5HT₁ agonist is beneficial in the treatment of conditions associated with pain and in alleviating the symptoms associated therewith.

The use of combinations of the present invention may give rise to an equivalent effect in the treatment of conditions associated with pain and in alleviating the symptoms associated therewith, or an increase in drug efficacy because synergy between compounds occurs. The use of combinations of the present invention may alternatively and/or additionally reduce side effects compared to administration of a single compound. Drug efficacy may be assessed using pain models such as carrageenan model (Guilbaud G. & Kayser V. Pain 28 (1987) 99-107) for acute inflammatory pain, FCA model (Freund's Complete Adjuvant) (Hay et al., Neuroscience Vol 78, No 3 pp843-850, 1997) for chronic inflammatory pain, or CCl model (Chronic Constriction Injury) (Bennett, G. J. & Xie. Y.K. (1988) Pain, 33: 87-107) for neuropathic pain.

It will be appreciated that reference to treatment is intended to include prophylaxis as well as the alleviation of established symptoms.

We provide the use of an adenosine A1 agonist or a pharmaceutically acceptable derivative thereof and a 5HT₁ agonist or a pharmaceutically acceptable derivative thereof for the manufacture of a medicament for the treatment of conditions associated with pain and the alleviation of symptoms associated thereof.

The combinations of the invention are useful as analgesics. They may be used to improve the condition of a host, typically of a human being, suffering from pain. They may be employed to alleviate pain in a host. Thus, the combinations of the invention may be used as a preemptive analgesic to treat acute pain such as muscculoskeletal pain, post operative pain and surgical pain, chronic pain such as chronic inflammatory pain (e.g. rheumatoid arthritis (RA) and osteoarthritis (OA), neuropathic pain (e.g. post herpetic neuralgia (PHN), trigeminal neuralgia, neuropathies associated with diabetes and sympathetically maintained pain) and pain associated with cancer and fibromyalgia. The combinations of the invention may also be used in the treatment or prevention of migraine and/or pain associated with migraine, tension headache and cluster headaches and pain associated with Functional Bowel Disorders (e.g. Irritable Bowel Syndrome), non cardiac chest pain and non ulcer dyspepsia.

Additionally, the combinations of the present invention exhibit analgesic and antiinflammatory activity and are therefore useful in a number of chronic inflammatory pain conditions such as OA, RA and neuropathic conditions such as fibromyalgia and PHN.

By pharmaceutically acceptable derivative is meant any pharmaceutically acceptable salt, solvate, ester or amide, or salt or solvate of such ester or amide, of the adenosine A1 agonist or the 5HT₁ agonist, or any other compound which upon administration to the recipient is capable of providing (directly or indirectly) the adenosine A1 agonist or the 5HT₁ agonist or an active metabolite or residue thereof.

Suitable physiologically acceptable salts according to the invention include acid addition salts formed with inorganic acids such as hydrochlorides, hydrobromides, phosphates and sulphates and with organic acids, for example tatrates, maleates, fumarates, succinates and sulphonates.

Suitable adenosine A1 agonists include adenosine, (2S, 3S, 4R, 5R)-2-(5 tert-butyl-[1,3,4]oxadiazol-2-yl)-S-[6-(4-chloro-2-fluoro-phenylamino)-purin-9-yl]-tetrahydro-furan-3,4-diol (synthesis as in Example 1), N-[1S, trans)-2-hydroxycyclopentyl]adenosine and N-(4-chloro-2-fluoro-phenyl)-5'-O-trifluoromethyl-adenosine. Particularly preferred are N-(4-Chloro-2-fluorophenyl)-5'-O-trifluoromethyl-adenosine and (2S, 3S, 4R, 5R)-2-(5 tert-butyl-[1,3,4]oxadiazol-2-yl)-5-[6-(4-chloro-2-fluoro-phenylamino)-purin-9-yl]-tetrahydrofuran-3,4-diol.

The compounds of WO99/67262 and physiologically acceptable salts or solvates thereof may be prepared by the processes described below. In the following description, the groups R¹, R² and R³ are as defined for compounds of formula (I) unless otherwise stated.

According to a first general process A, a compound of formula (I) may be prepared by reacting a compound of formula (II) 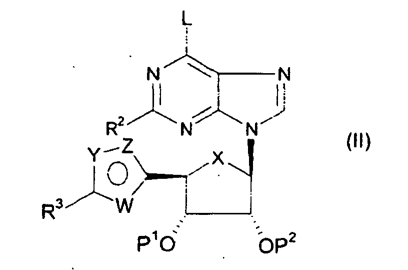 wherein L represents a leaving group such as a halogen atom (e.g. chlorine), or a linker group capable of binding to a solid phase polymeric support (e.g. a polystyrene resin) and for example may be -SO₂C₁₋₄alkylene and P¹ and P² represent hydrogen, C₁₋₆ straight chain or branched alkyl or a suitable protecting group (e.g. acetyl or a protecting group wherein P¹ and P² together form an alkylidine group) with a compound of formula R¹NH₂ or a salt thereof under basic conditions. The 4'-heterocycle group substituent may be protected if required.

Compounds of formula (II) may be used to produce compounds of formula (I) directly by reaction with the group R¹NH₂ either in the absence or presence of a solvent such as an alcohol (e.g. a lower alkanol such as isopropanol, t-butanol or 3-pentanol), an ether (e.g. tetrahydrofuran or dioxan), a substituted amide (e.g. dimethylformamide), a halogenated hydrocarbon (e.g. chloroform), an aromatic hydrocarbon (e.g. toluene), dimethyl sulfoxide (DMSO) or acetonitrile, preferably at an elevated temperature (e.g. up to the reflux temperature of the solvent), in the presence of a suitable acid scavanger, for example, inorganic bases such as sodium, cesium or potassium carbonate, or organic bases such as triethylamine, diisopropylethylamine or pyridine, optionally in the presence of a palladium catalyst (e.g. palladium acetate) and phosphine ligand (e.g. R-(+)-2,2'-bis(dipheny)phosphino)-1-1' binaphthyl).

Optionally, where at least one of Y, Z and W is N, alkylation may be carried out on a N atom at Y, Z or W at any appropriate stage in the synthesis.

The above reactions may be preceded or followed where appropriate by in situ removal of the P¹ and P² protecting groups. For example when P¹ and P² represent acetyl, this may be effected with an amine such as ammonia or tert-butylamine in a solvent such as methanol or when P¹ and P² represent an alkylidine by acid hydrolysis, e.g. with trifluoroacetic acid (TFA). Interconversion of P¹ and P² protecting groups may occur at any stage in the preparation of the compounds of formula (II), for example when P¹ and P² represent acetyl, compounds of formula (II) may be prepared from compounds wherein P¹ and P² together represent an alkylidine protecting group by acid catalysed removal of the alkylidine protecting group, e.g. with hydrogen chloride in methanol followed by in situ acylation, for example with acetic anhydride in the presence of a base such as pyridine, in a solvent such as dichloromethane.

Otherwise, interconversion of P¹ and P² protecting groups may occur at any stage during the preparation of compounds of formula (II).

It will be apparent to persons skilled in the art that in the preparation of compounds of formula (II) or (I) the 4'-heterocycle may be formed at any stage. For example, heterocycles may be prepared from carboxylic acid or acetylene starting materials before the addition of the purine ring (see Schemes 1, 1 a and 2) or heterocycles may be formed after the addition of the purine ring (see Scheme 3).

Compounds of formula (II) where X = O may be prepared by reacting compounds of formula (III) 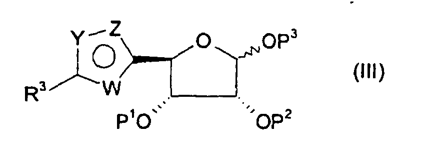 wherein P³ represents a suitable protecting group, for example acetyl, or a substituent such as C₁₋₃ alkyl, and P¹, P² and R³ are as defined above, with compounds of formula (IV) 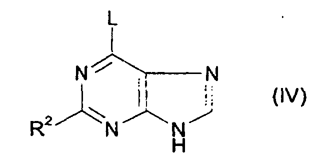 wherein L and R² are as defined above.

The reaction is conveniently carried out in a suitable solvent, such as acetonitrile in the presence of a silylating agent such as trimethylsilyl trifluoromethane sulfonate and a base such as diazabicyclo[5.4.0]undec-7-ene (DBU). Alternatively the compound of formula (IV) may first be silylated with a suitable silylating agent e.g. hexamethyldisilazane followed by reaction of the silylated intermediate with a compound of formula (III) and a suitable Lewis acid, e.g. trimethylsilyl trifluoromethanesulfonate in a suitable solvent such as acetonitrile.

Compounds of formula (IV) are either known in the art or may be prepared from known compounds using methods analogous to those used to prepare the known compounds of formula (IV).

As described above, the compounds of formula (III) may be prepared from alternative protected compounds by replacement of the alternate P¹ and P² protecting groups with other P¹ and P² groups. These represent an exchanging of one protecting group for another and will be apparent to those skilled in the art. Compounds of formula (III) may be made for example by the following syntheses:

Compounds of formula (III) may be prepared, for example when the heterocycle defined by W, Y and Z hereinabove represents an isoxazole (optionally substituted) by the following reaction schemes.

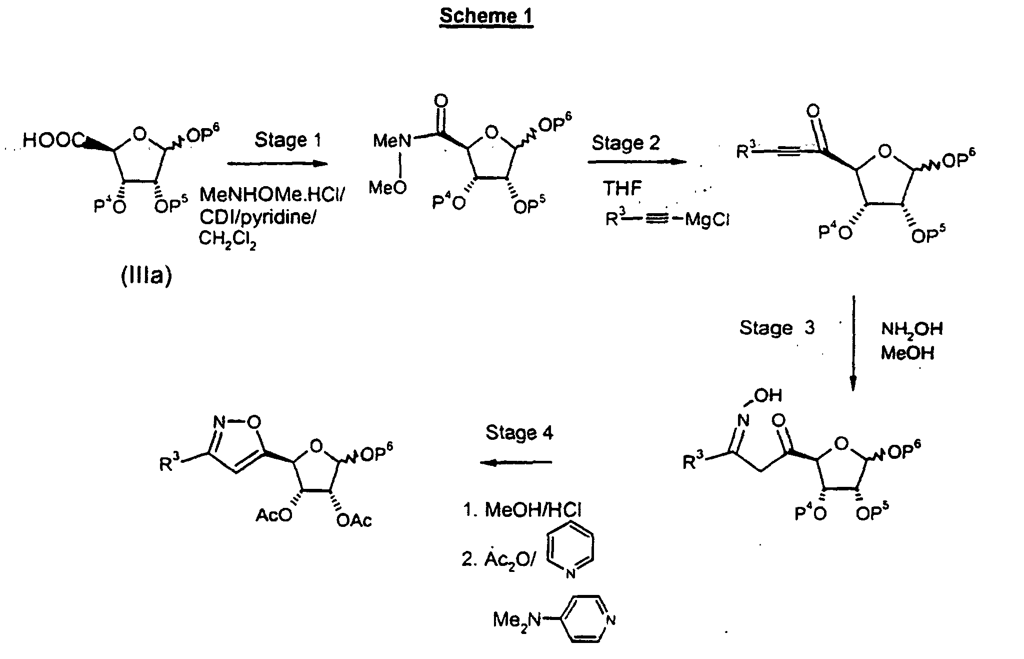

General conditions for Stages 1-4 will be known to persons skilled in the art. It will also be appreciated that the reagents and conditions set out in Scheme 1 are example conditions and alternative reagents and conditions for achieving the same chemical transformation may be known to persons skilled in the art. P⁴ and P⁵ together represent alkylidine protecting group(s). P⁶ represents C_{1-4.} alkyl. R³ is as previously defined.

Although scheme 1 shows the preparation of compounds of formula (III) where the heterocycle moiety is an isoxazole it would be apparent to a person skilled in the art that other standard methods could be employed to produce compounds of formula (III) with other heterocycles from carboxylic acid starting materials.

An alternative method for synthesis of compounds of formula (III) is shown in Scheme 1a.

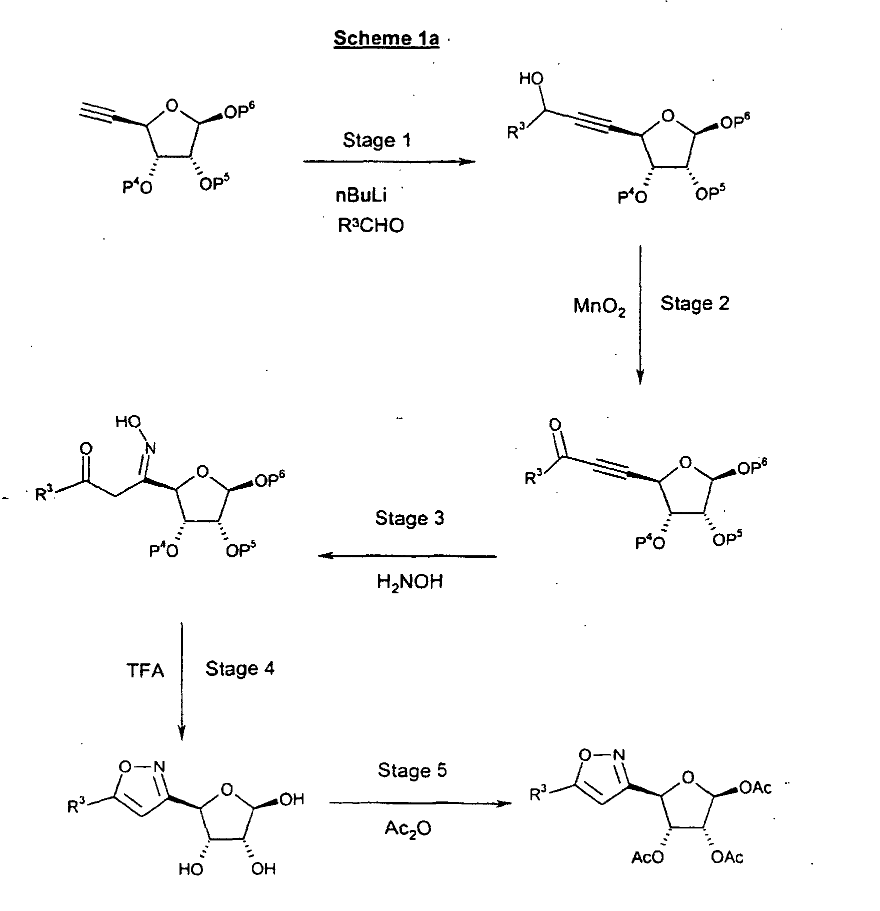

General conditions for Stages 1-5 in Scheme 1a will be known to persons skilled in the art. R³, P⁴, P⁵ and P⁶ are as previously defined.

Scheme 2 represents a method of preparing compounds of formula (III) when Y = N, Z = NH, W = CH and R³ = H or tautomers thereof. P¹, P² and P⁶ are as previously defined.

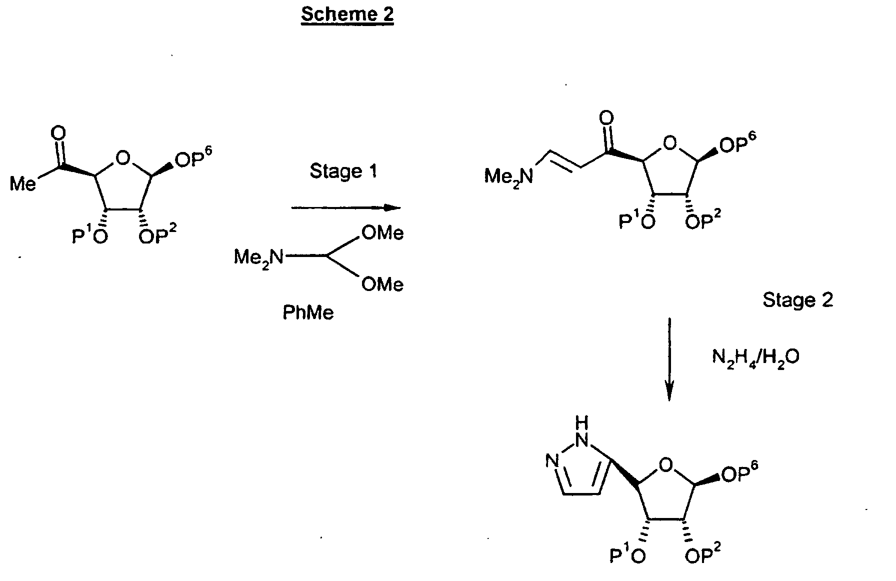

A further process (B) comprises converting a compound of formula (I) into a different compound of formula (I) by modifying the R¹, R² and/or R³ groups therein.

Compounds of the formula R¹NH₂ are either known compounds or may be prepared from known compounds using conventional procedures.

Specific optical isomers of a compound of formula (I) may be obtained by conventional methods for example, by synthesis from an appropriate asymmetric starting material using any of the processes described herein, or where appropriate by separation of a mixture of isomers of a compound of formula (I) by conventional means e.g by fractional crystallisation or chromatography.

According to a third process (C), compounds of formula (I) may be prepared from compounds of formula (V) or (VI): 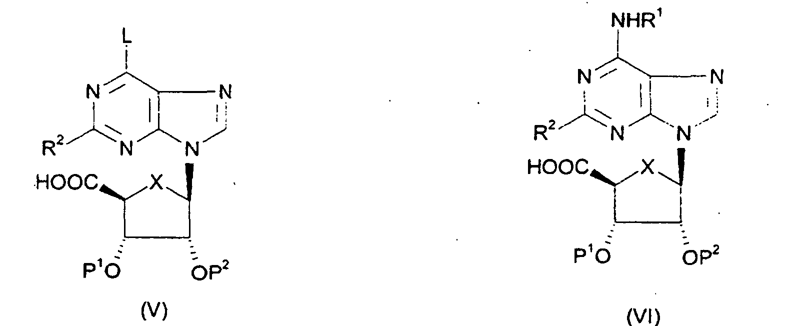 where R¹, R²,X, L, P¹ and P² represent groups as previously defined.

Also compounds of formula (VI) may be prepared from compounds of formula (V) by analogous methods to those described in process (A) above.

Synthesis of the compounds of formulae (I) from the corresponding acids of formulae (V) and (VI) will be apparent to a skilled person using conventional synthetic techniques.

As an example, when W = O, Y = N and Z = N in formula (I) above thus defining a 1,3,4 oxadiazole, the synthesis is according to reaction scheme 3. J represents a leaving group L as previously defined, or a NHR¹ group. R², X, P¹ and P² are as previously defined.

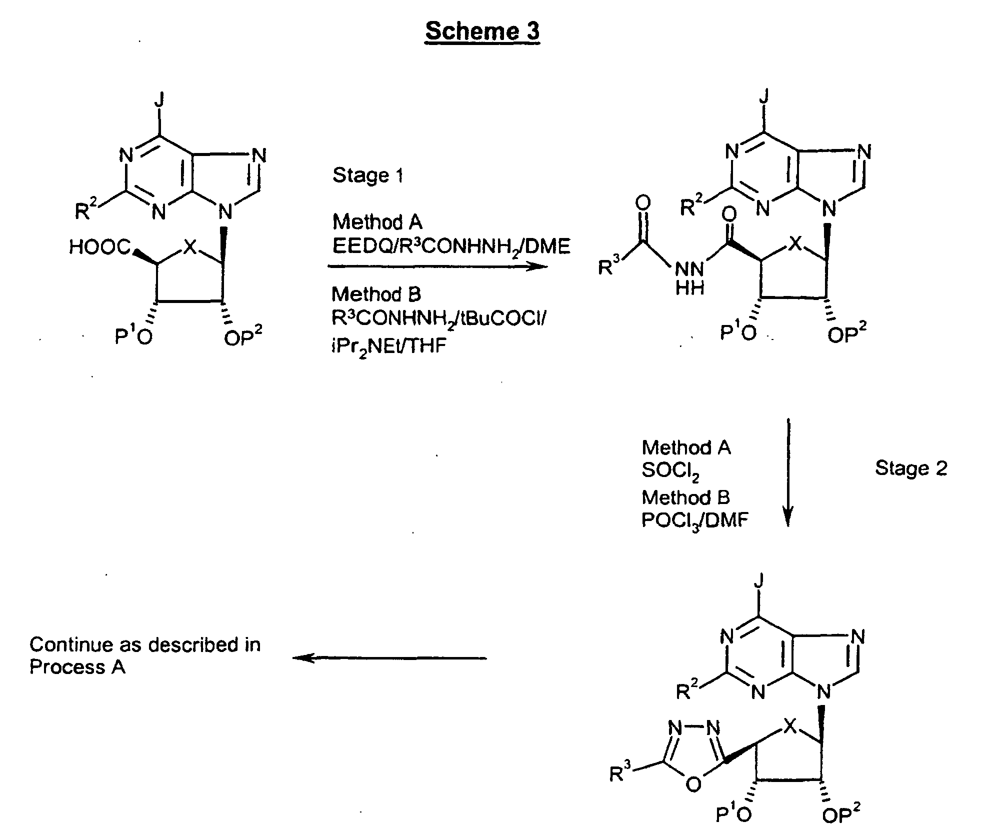

Compounds of formula (I) where Z=O, Y=N and W=N (thus defining a 1,3,4-oxadiazole) may be prepared from compounds of formula (V) or (VI) by a first process involving activation of the carboxyl group on the compound of formula (V) or (VI) followed by reaction with an amidoxime of formula HO-N=C(R³)NH₂ in a solvent such as tetrahydrofuran or chloroform, in the presence of a base such as pyridine or di-isopropylethylamine, followed by cyclisation at a temperature of 20°C-150°C in a solvent such as toluene, tetrahydrofuran (THF) or chloroform. Methods of carboxyl activation include reaction with an acid chloride, such as pivaloyl chloride, or an acid anhydride in the presence of a base such as a tertiary amine, for example di-isopropylethylamine, or with thionyl chloride in dimethylformamide (DMF). Activating agents used in peptide chemistry such as 2-ethoxy-1-ethoxycarbonyl-1,2-dihydroquinoline (EEDQ) or 1-hydroxybenzotriazole and 1-(3-dimethylaminopropyl)-3-ethyl-carbodiimide hydrochloride, may also be used. Hydroxyl protecting groups may be removed under conditions known to those practising in the art. For example, the acetonide group may be removed by treatment with an acid (at a temperature of 0°C-150°C) such as trifluoroacetic acid suitably at 0-20°C or acetic acid suitably at 50-150°C.

A preferred compound is (2S, 3S, 4R, 5R)-2-(5-tert-butyl-[1,3,4]oxadiazol-2-yl)5-6[6-(4-chloro-2-fluoro-phenylamino)purin-9-yl]tetrahydro-furan-3,4-diol.

Therefore in a preferred aspect of the invention there is provided the use of (2S, 3S, 4R, 5R)-2-(5 tert-butyl-[1,3,4]oxadiazol-2-yl)-5-[6-(4-chloro-2-fluorophenylamino)-purin-9-yl]-tetrahydro-furan-3,4-diol or a pharmaceutically acceptable derivative thereof and a 5HT₁ agonist or a pharmaceutically acceptable derivative thereof in treating conditions associated with pain and alleviating the symptoms associated therewith in a mammal, including man.

It will be appreciated that the present invention relates to the use of an adenosine A1 agonist in conjunction with any compound having 5HT₁ agonist activity known in the art.

A variety of 5HT₁ agonists have been described in the art, for example those mentioned in the following published British Patent Specifications Nos. 2082175, 2081717, 2083463, 2124210, 2150932, 2162522, 2168347, 2168973, 2185020, 2186874, 2191488, 2208646, 2289464, 2289465, 2286185, published European Patent Specifications Nos. 147107, 237678, 242939, 244085, 225726, 254433, 303506, 313397, 354777, 382570, 464558, 506363, 506369, 450238, 451022, 451008, 478954, 438230, 494774, 497512, 501568, 580539, 581538, 586866, 590970, 590971, 603432, 610134, 620222, 620223, 644187, 641787, 645385, 648767, 666258, 668273, 683155, 700905, 703229, 707007, 708102, 712837, 714894, 714896, 729958, 733628, 736525, 747353, 749962, 755932, 768301, 810220, and published International patent application Nos. WO92/11013, W092/11014, WO92/06973, WO93/00086, WO92/13856, WO93/00094, WO91/18897, WO93/00333, WO94/02460, WO94/02477, WO94/03446, WO94/06789, WO94/10171, WO94/11363, WO94/11356, WO94/13620, WO94/14770, WO94/14771, WO94/14772, WO94/14773, WO94/14779, WO94/15930, WO94/18193, WO94/20466, WO94/21610, WO94/21611, WO94/21619, WO94/24127, WO94/29293, WO95/01334, WO95/01965, WP95/05366, WO95/05381, WO95/05383, WO95/06636, WO95/11903, WO95/20588, WO95/21166, WO95/21167, WO95/28933, WO95/32196, WO96/04269, WO96/04274, WO96/06638, WO96/06846, WO96/09288, WO96/11195, WO96/11685, WO96/11923, WO96/11930, WO96/12721, WO96/12713, WO96/16056, WO96/16949, WO96/16961, WO96/17831, WO96/17842, WO96/23784, WO96/23785, WO96/23789, WO96/24596, WO96/29075, WO96/39133, WO96/41802, WO97/03068, WO97/08159, WO97/11695, WO97/11945, WO97/11946, WO97/13512, WO97/16446, WO97/17337, WO97/17338, WO97/17343, WO97/18201, WO97/18202, WO97/18203, WO97/18204, WO97/19073, WO97/38692, WO97/42189, WO97/43281, WO97/45426, WO95/14004, WO98/12183, WO98/14433 and WO98/15545. The above applications also describe, in relation to the 5HT₁ agonists they disclose, both suitable methods for their preparation and doses for their administration.

5HT₁ agonists have also recently been classified as 5HT_{1B/1D} agonists and the terms "5HT₁ agonist" and "5HT_{1B/1D} agonist" may be used interchangeably herein.

Suitable 5HT₁ like receptor agoinsts for use according to the invention include: sumatriptan, naratriptan, zolmitriptan, eletriptan, rizatriptan, frovatriptan, almotriptan, alniditan, dihydroergotamine, donitriptan, PNU-142633, ALX-0646, LY334370, U1092291, IS159 and PNY142633.
Further suitable 5HT₁ agonists for use according to the invention include: sumatriptan, naratriptan, zolmitriptan, eletriptan, rizatriptan, frovatriptan, almotriptan, afniditan, ALX-0646, LY334370, U1092291, IS159 and PNY142633. Naratriptan and sumatriptan are preferred with sumatriptan being particularly preferred. A preferred form of sumatriptan is the succinate salt, particularly the 1:1 succinate.

Therefore according to a further aspect of the invention there is provided the use of an adenosine A1 agonist or a pharmaceutically acceptable derivative thereof and sumatriptan or a pharmaceutically acceptable derivative thereof in treating conditions associated with pain and alleviating the symptoms associated therewith in a mammal, including man.

A particular preferred combination of the invention is (2S, 3S, 4R, 5R)-2-(5 tert-butyl-[1,3,4]oxadiazol-2-yl)-5-[6-(4-chloro-2-fluoro-phenylamino)-purin-9-yl]-tetrahydro-furan-3,4-diol or a pharmaceutically acceptable derivative thereof and sumatriptan or a pharmaceutically acceptable derivative thereof.

Compounds for use according to the invention may be administered simultaneously or sequentially and, when administration is sequential, either the adenosine A1 agonist or the 5HT₁ agonist may be administered first. When administration is simultaneous, the combination may be administered either in the same or different pharmaceutical composition.

Compounds for use according to the invention may be administered as the raw material but the active ingredients are preferably provided in the form or pharmaceutical formulations.

The active ingredients may be used either as separate formulations or as a single combined formulation. When combined in the same formulation it will be appreciated that the two compounds must be stable and compatible with each other and the other components of the formulation. Therefore, pharmaceutical formulations comprising a combination as defined above together with a pharmaceutically acceptable diluent or carrier comprise a further aspect of the invention. When formulated separately they may be provided in any convenient formulation, conveniently in such manner as are known for such compounds in the art.

Accordingly in a further aspect of the invention we provide a pharmaceutical composition which comprises a adenosine A1 agonist or a pharmaceutically acceptable derivative thereof and the 5HT₁ agonist or a pharmaceutically acceptable derivative thereof formulated for administration by any convenient route. Such compositions are preferably in a form adapted for use in medicine, in particular human medicine, and can conveniently be formulated in conventional manner using one or more pharmaceutically acceptable carriers or excipients.

The formulations include those suitable for oral, parenteral (including subcutaneous e.g. by injection or by depot tablet, intradermal, intrathecal, intramuscular e.g. by depot and intravenous), rectal and topical (including dermal, buccal and sublingual) or in a form suitable for administration by inhalation or insufflation administration, although the most suitable route may depend upon for example the condition and disorder of the recipient. The formulations may conveniently be presented in unit dosage form and may be prepared by any of the methods well known in the art of pharmacy. All methods include the step of bringing into association the compounds ("active ingredient") with the carrier which constitutes one or more accessory ingredients. In general the formulations are prepared by uniformly and intimately bringing into association the active ingredient with liquid carriers or finely divided solid carriers or both and then, if necessary, shaping the product into the desired formulation. Preferably such compositions will be formulated for oral administration. It will be appreciated that when the two active ingredients are administered independently, each may be administered by different means.

Formulations suitable for oral administration may be presented as discrete units such as capsules, cachets or tablets (e.g. chewable tablets in particular for paediatric administration) each containing a predetermined amount of the active ingredient; as a powder or granules; as a solution or a suspension in an aqueous liquid or a non-aqueous liquid; or as an oil-in-water liquid emulsion or a water-in-oil liquid emulsion. The active ingredient may also be presented as a bolus, electuary or paste.

A tablet may be made by compression or moulding, optionally with one or more accessory ingredients. Compressed tablets may be prepared by compressing in a suitable machine the active ingredient in a free-flowing form such as a powder or granules, optionally mixed with a other conventional excipients such as binding agents, (for example, syrup, acacia, gelatin, sorbitol, tragacanth, mucilage of starch, polyvinylpyrrolidone) or hydroxymethyl cellulose or hydroxymethyl cellulose fillers (for example, lactose, sugar, microcrystalline cellulose, maize-starch, calcium phosphate or sorbitol), lubricants (for example, magnesium stearate, stearic acid, talc, polyethylene glycol or silica), disintegrants (for example, potato starch or sodium starch glycollate) or wetting agents, such as sodium lauryl sulfate. Moulded tablets may be made by moulding in a suitable machine a mixture of the powdered compound moistened with an inert liquid diluent. The tablets may optionally be coated or scored and may be formulated so as to provide slow or controlled release of the active ingredient therein. The tablets may be coated according to methods well-known in the art.

Alternatively, the compounds of the present invention may be incorporated into oral liquid preparations such as aqueous or oily suspensions, solutions, emulsions, syrups or elixirs, for example. Moreover, formulations containing these compounds may be presented as a dry product for constitution with water or other suitable vehicle before use. Such liquid preparations may contain conventional additives such as suspending agents such as sorbitol syrup, methyl cellulose, glucose/sugar syrup, gelatin, hydroxyethylcellulose, carboxymethyl cellulose, aluminum stearate gel or hydrogenated edible fats; emulsifying agents such as lecithin, sorbitan mono-oleate or acacia; non-aqueous vehicles (which may include edible oils) such as almond oil, fractionated coconut oil, oily esters, propylene glycol or ethyl alcohol; and preservatives such as methyl or propyl p-hydroxybenzoates or sorbic acid. Such preparations may also be formulated as suppositories, e.g., containing conventional suppository bases such as cocoa butter or other glycerides.

Formulations for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents.

The formulations may be presented in unit-dose or multi-dose containers, for example sealed ampoules and vials, and may be stored in a freeze-dried (lyophilised) condition requiring only the addition of a sterile liquid carrier, for example, water-for-injection, immediately prior to use. Extemporaneous injection solutions and suspensions may be prepared from sterile powders, granules and tablets of the kind previously described.

Formulations for rectal administration may be presented as a suppository with the usual carriers such as cocoa butter, hard fat or polyethylene glycol.

Formulations for topical administration in the mouth, for example buccally or sublingually, include lozenges comprising the active ingredient in a favoured basis such as sucrose and acacia or tragacanth, and pastilles comprising the active ingredient in a basis such as gelatin and glycerin or sucrose and acacia.

For topical administration to the epidermis, the compounds may be formulated as creams, gels, ointments or lotions or as a transdermal patch.

The compounds may also be formulated as depot preparations. Such long acting formulations may be administered by implantation (for example subcutaneously or intramuscularly) or by intramuscular injection. Thus, for example, the compounds may be formulated with suitable polymeric or hydrophobic materials (for example as an emulsion in an acceptable oil) or ion exchange resins, or as sparingly soluble derivatives, for example, as a sparingly soluble salt.

For intranasal administration the compounds of the invention may be used, for example as a liquid spray, as a powder or in the form of drops.

For administration by inhalation the compounds according to the invention are conveniently delivered in the form of an aerosol spray presentation from pressurised packs or a nebuliser, with the use of a suitable propellant, e.g. 1,1,1,2-trifluoroethane (HFA 134A) and 1,1,1,2,3,3,3, - heptapropane (HFA 227), carbon dioxide or other suitable gas. In the case of a pressurised aerosol the dosage until may be determined by providing a valve to deliver a metered amount. Capsules and cartridges of e.g. gelatin for use in an inhaler or insufflator may be formulated containing a powder mix of a compound of the invention and a suitable powder base such as lactose or starch.

In addition to the ingredients particularly mentioned above, the formulations may include other agents conventional in the art having regard to the type of formulation in question, for example those suitable for oral administration may include flavouring agents.

It will be appreciated by those skilled in the art that reference herein to treatment extends to prophylaxis as well as the treatment of established diseases or symptoms. Moreover, it will be appreciated that the amount of a compound of the invention required for use in treatment will vary with the nature of the condition being treated and the age and the condition of the patient and will be ultimately at the discretion of the attendant physician or veterinarian. In general, however, doses employed for adult human treatment will typically be in the range of 0.02-5000 mg per day, preferably 1-1500 mg per day. The desired dose may conveniently be presented in a single dose or as divided doses administered at appropriate intervals, for example as two, three, four or more sub-doses per day. The formulations according to the invention may contain between 0.1-99% of the active ingredient, conveniently from 30-95% for tablets and capsules and 3-50% for liquid preparations.

Pharmaceutical compositions according to the invention may be prepared by conventional techniques. When combined in the same formulation for example, the adenosine A1 agonist or a pharmaceutically acceptable derivative thereof and 5HT₁ agonist or a pharmaceutically acceptable derivative thereof may be admixed together, if desired, with suitable excipients. Tablets may be prepared, for example, by direct compression of such a mixture. Capsules may be prepared, for example by filling the blend together with suitable excipients into gelatin capsules, using a suitable filling machine.

Compositions for use according to the invention may, if desired, be presented in a pack or dispenser device which may contain one or more unit dosage forms containing the active ingredients. The pack may, for example, comprise metal or plastic foil, such as a blister pack. Where the compounds are intended for administration as two separate compositions these may be presented, for example, in the form of a twin pack.

Pharmaceutical compositions may also be prescribed to the patient in "patient packs" containing the whole course of treatment in a single package, usually a blister pack. Patient packs have an advantage over traditional prescriptions, where a pharmacists divides a patients supply of a pharmaceutical from a bulk supply, in that the patient always has access to the package insert contained in the patient pack, normally missing in traditional prescriptions. The inclusion of a package insert has been shown to improve patient compliance with the physicians instructions.

It will be understood that the administration of the combination of the invention by means of a single patient pack, or patients packs of each composition, including a package insert directing the patient to the correct use of the invention is a desirable additional feature of this invention.

According to a further aspect of the invention there is provided a patient pack comprising at least one active ingredient, of the combination according to the invention and an information insert containing directions on the use of the combination of the invention.

According to another aspect the invention provides a double pack comprising in association for separate administration an adenosine A1 agonist or a pharmaceutically acceptable derivative thereof and- a 5HT₁ agonist or pharmaceutically acceptable derivative thereof.

It will be appreciated that the dose at which the adenosine A1 agonist and the 5HT₁ agonist is being administered will depend on the age and condition of the patient and the frequency and route of administration and will be at the ultimate discretion of the attendant physician. The active ingredients may conveniently be presented in unit dose form.

A proposed dose of adenosine A1 agonist and 5HT₁ agonist agonist for administration to man (of approximately 70kg body weight) may conveniently be administered at doses within the normal range taught in the art at which the compounds are therapeutically effective.

For example, a proposed dose of the adenosine A1 agonist for use according to the invention is 0.1mg to 2g, preferably 1mg to 2g, more preferably 1 to 100mg per unit dose, expressed as the weight of free base. The unit dose may be administered in single or divided doses, for example, from 1 to 4 times per day.

For example a proposed dose of 5HT₁ agonist for use according to the invention is 0.001 mg to 500mg. The unit dose may be administered in single or divided doses for example from 1 to 4 times per day.

### Example 1

### Intermediate 1

### (3aS,4S,6R,6aR)-6-(6-Chloro-purin-9-yl)-2,2-dimethyl-tetrahydro-furo[3,4-d][1,3] dioxole-4-carboxylic acid N'-(2,2-dimethyl-propionyl)-hydrazide

(3aS,4S,6R,6aR)-6-(6-Chloro-purin-9-yl)-2,2-dimethyl-tetrahydro-furo[3,4-d][1,3] dioxole-4-carboxylic acid (2.5g) suspended in 1,2-dimethoxymethane (100ml) was treated with 2,2-dimethyl-propionic acid hydrazide (1.1g) and 2-ethoxy-1-ethoxycarbonyl-1,2-dihydroquinoline (EEDQ), and the mixture heated under reflux for 16h. The mixture was poured into aqueous citric acid (250ml) and extracted with ethyl acetate; the organic layers were washed with citric acid and brine, dried (MgSO₄) and evaporated in vacuo to give the crude product. Purification by flash chromatography on silica gel (Biotage cartridge), eluting with ethyl acetate:cyclohexane 65:35, gave the title compound as a white solid (1.92g).
LC/MS (System B): Rₜ 2.49 min
Mass spectrum *m*/*z* 439 [MH⁺].

### Intermediate 2

### 9-[6S-(5-tert-Butyl-[1,3,4]oxadiazol-2-yl)-2,2-dimethyl-tetrahydro-(3aR,6aS)-furo[3,4-d][1,3]dioxol-4R-yl]-6-chloro-9H-purine

(3aS,4S,6R,6aR)-6-(6-Chloro-purin-9-yl)-2,2-dimethyl-tetrahydro-furo[3,4-d][1,3]dioxole-4-carboxylic acid N'-(2,2-dimethyl-propionyl)-hydrazide (1.5g) was dissolved in thionyl chloride (15ml) and the solution irradiated in a microwave oven at 150W power for 7 min. The excess thionyl chloride was evaporated in vacuo to give the crude product which was dissolved in dry acetonitrile (6ml) and heated under reflux for 3h. The solvent was evaporated and the residue purified by flash chromatography on silica gel, eluting with ethyl acetate:cyclohexane 35:65 - 40:60, to give the title compound as a white solid (0.645g).
LC/MS (System B): Rₜ 2.86min
Mass spectrum *m*/*z* 421 [MH⁺].

### Intermediate 3

### 9-{(3aR,4R,6S,6aR)-6-[5-(tert-butyl)-1,3,4-oxadiazol-2-yl]-2,2-dimethyltetrahydrofuro[3,4-d][1,3]dioxol-4-yl}-N-(4-chloro-2-fluorophenyl)-9H-purin-6-amine

9-[6S-(5-tert-Butyl-[1,3,4]oxadiazol-2-yl)-2,2-dimethyl-tetrahydro-(3aR,6aS)-furo[3,4-d][1,3]dioxol-4R-yl]-6-chloro-9H-purine (2.8g) was treated with 4-chloro-2-fluoro-aniline (4.48ml), palladium acetate (146mg) and (R)-2,2'-bis(diphenylphosphino)-1,1'-binaphthyl (620mg) in dry toluene (34ml) and the mixture stirred at room temperature for 5 mins (reaction carried out in seven portions). Caesium carbonate (3.08g, in seven portions) was added, and the mixtures heated at 86-96° C for 16h. The mixtures were combined and partitioned between water (200ml) and dichloromethane (3x120ml). The organic layers were washed with brine, dried (MgSO₄) and evaporated *in vacuo* to give a brown oil (8.7g). Purification by chromatography on silica gel, eluting with ethyl acetate:cyclohexane 30:70 gave an off-white solid (2.35g).
LC/MS (System C) Rₜ = 3.41 min
Mass Spectrum *m*/*z* 530 [MH⁺]

### (2S,3S,4R,5R)-2-(5-tert-Butyl-[1,3,4]oxadiazol-2-yl)-5-[6-(4-chloro-2-fluoro-phenylamino)-purin-9-yl]-tetrahydro-furan-3,4-diol

9-{(3aR,4R,6S,6aR)-6-[5-(tert-butyl)-1,3,4-oxadiazol-2-yl]-2,2-dimethyltetrahydrofuro[3,4-d][1,3]dioxol-4-yl}-N-(4-chloro-2-fluorophenyl)-9H-purin-6-amine (2.35g) was dissolved in trifluoroacetic acid (20ml) and water (2ml) with ice bath cooling, and the mixture allowed to stand at 4°C for 17h. The mixture was poured slowly into ice cold saturated aqueous sodium bicarbonate (400ml) and extracted with ethyl acetate (3x200ml). The organic layers were washed with brine, dried (MgSO₄) and evaporated *in vacuo* to give the title compound as a buff solid (2.30g).
LC/MS (System C) Rₜ = 3.04 min.
Mass Spectrum *m*/*z* 490 [MH⁺]

## Claims

1. Use of an adenosine A1 agonist or a pharmaceutically acceptable derivative thereof and 5HT₁ agonist or a pharmaceutically acceptable derivative thereof for the manufacture of a medicament for the treatment of conditions associated with pain and the alleviation of symptoms associated thereof.

2. A use according to claim 1 wherein the condition associated with pain is acute pain, chronic pain, inflammatory pain, neuropathic pain, pain associated with migraine, tension headaches, cluster headaches or functional bowel disorder.

3. A use according to claim 1 or claim 2 wherein the adenosine A1 agonist is selected from adenosine, N-(4-chloro-2-fluoro-phenyl)-5'-O'-trifluoromethyl-adenosine, N-[(1S, trans)-2-hydroxycyclopentyl]adenosine and (2S, 3S, 4R, 5R)-2-(5-tert-butyl-[1,3,4]oxadiazol-2-yl)-5-[6-(4-chloro-2-fluoro-phenylamino)-purin-9-yl]-tetrahydro-furan-3,4-diol, or a pharmaceutically acceptable derivative thereof.

4. A use according to claim 3 wherein the adenosine A1 agonist is (2S, 3S, 4R, 5R)-2-(5-tert-butyl-[1,3,4]oxadiazol-2-yl)-5-[6-(4-chloro-2-fluoro-phenylamino)-purin-9-yl]-tetrahydro-furan-3,4-diol or a pharmaceutically acceptable derivative thereof.

5. A use according to any one of claims 1 to 4 wherein the 5HT₁ agonist is: sumatriptan, naratriptan, zolmitriptan, eletriptan, rizatriptan, frovatriptan, almotriptan, alniditan, dihydroergotamine, donitriptan, (1S)-1-(2-(4-(4-(aminocarbonyl)phenyl)-1-piperazinyl)ethyl-3,4-dihydromethyl-1H-2-benzopyran-6-carboxamide) (PNU-142633), N,N-dimethyl-5-(1,2,3,6-tetrahydro-1-methyl-4-pyridinyl)-1H-indole-3-ethanamine (ALX-0646), 4-fluoro-N-[3-(1-methyl-4-piperidinyl)-1H-indol-5-yl]benzamide (LY334370), or N-(((3-(2-aminoethyl)1H-indol-5-yl)oxy)acetyl)-L-tyrosyl glycinamide (IS159) or a pharmaceutically acceptable derivative thereof.

6. A use according to claim 5 wherein the 5HT₁ agonist is sumatriptan, naratriptan, zolmitriptan, eletriptan, rizatriptan, frovatriptan, almotriptan, alniditan, N,N-dimethyl-5-(1,2,3,6-tetrahydro-1-methyl-4-pyridinyl)-1H-indole-3-ethanamine (ALX-0646), 4-fluoro-N-[3-(1-methyl-4-piperidinyl)-1H-indol-5-yl)benzamide (LY334370) or N-(((3-(2-aminoethyt)-1H-indol-5-yl)oxy)acetyl)-L-tyrosyl glycinamide (IS159) or a pharmaceutically acceptable derivative thereof.

7. A use according to claim 5 or claim 6 wherein the 5HT₁ agonist is sumatriptan or naratriptan or a pharmaceutically acceptable derivative thereof.

8. A use according to claim 1 or claim 2 wherein the adenosine A1 agonist is (2S, 3S, 4R, 5R)-2-(5-tert-butyl-[1,3,4]oxadiazol-2-yl)-5-[6-(4-chloro-2-fluoro-phenytamino)-purin-9-yl]-tetrahydro-furan-3,4-diol and the 5HT₁ agonist is sumatriptan or naratriptan or a pharmaceutically acceptable derivative thereof.

9. A pharmaceutical composition which comprises an adenosine A1 agonist or a pharmaceutically acceptable derivative thereof and a 5HT₁ agonist or a pharmaceutically acceptable derivative thereof.

10. A pharmaceutical composition according to claim 9 adapted for oral administration.

11. A patient pack comprising an adenosine A1 agonist or a pharmaceutically acceptable derivative thereof and a 5HT₁ agonist or a pharmaceutically acceptable derivative thereof.

## Patentansprüche

1. Verwendung eines Adenosin-A1-Agonisten oder eines pharmazeutisch verträglichen Derivats davon und eines 5HT₁-Agonisten oder eines pharmazeutisch verträglichen Derivats davon zur Herstellung eines Medikaments zur Behandlung von mit Schmerz verbundenen Zuständen und zur Linderung der damit verbundenen Symptome.

2. Verwendung gemäß Anspruch 1, wobei der mit Schmerz verbundene Zustand akuter Schmerz, chronischer Schmerz, Entzündungsschmerz, neurogener Schmerz, mit Migräne verbundener Schmerz, Spannungskopfschmerzen, Cluster-Kopfschmer oder funktionelle Darmstörung ist.

3. Verwendung gemäß Anspruch 1 oder Anspruch 2, wobei der Adenosin-A1-Agonist ausgewählt ist aus Adenosin, N-(4-Chlor-2-fluorphenyl)-5'-O'-trifluormethyladenosin, N-[(1S,trans)-2-Hydroxycyclopentyl]adenosin und (2S, 3S, 4R, 5R)-2-(5-tert.-Butyl-[1,3,4]oxadiazol-2-yl)-5-[6-(4-chlor-2-fluorphenylamino)purin-9-yl]tetrahydrofuran-3,4-diol oder einem pharmazeutisch verträglichen Derivat davon.

4. Verwendung gemäß Anspruch 3, wobei der Adenosin-A1-Agonist (2S, 3S, 4R, 5R)-2-(5-tert.-Butyl-[1,3,4]oxadiazol-2-yl)-5-[6-(4-chlor-2-fluorphenylamino)purin-9-yl]tetrahydrofuran-3,4-diol oder ein pharmazeutisch verträgliches Derivat davon ist.

5. Verwendung gemäß einem der Ansprüche 1 bis 4, wobei der 5HT₁-Agonist Sumatriptan, Naratriptan, Zolmitriptan, Eletriptan, Rizatriptan, Frovatriptan, Almotriptan, Alniditan, Dihydroergotamin, Donitriptan, (1S)-1-(2-(4-(4-(Aminocarbonyl)phenyl)-1-piperazinyl)ethyl-3,4-dihydromethyl-1H-2-benzopyran-6-carboxamid (PNU-142633), N,N-Dimethyl-5-(1,2,3,6-tetrahydro-1-methyl-4-pyridinyl)-1H-indol-3-ethanamin (ALX-0646), 4-Fluor-N-[3-(1-methyl-4-piperidinyl)-1H-indol-5-yl]benzamid (LY334370) oder N-(((3-(2-Aminoethyl)-1H-indol-5-yl)oxy)acetyl)-L-tyrosylglycinamid (IS159) oder ein pharmazeutisch verträgliches Derivat davon ist.

6. Verwendung gemäß Anspruch 5, wobei der 5HT₁-Agonist Sumatriptan, Naratriptan, Zolmitriptan, Eletriptan, Rizatriptan, Frovatriptan, Almotriptan, Alniditan, N,N-Dimethyl-5-(1,2,3,6-tetrahydro-1-methyl-4-pyridinyl)-1H-indol-3-ethanamin (ALX-0646), 4-Fluor-N-[3-(1-methyl-4-piperidinyl)-1H-indol-5-yl]benzamid (LY334370) oder N-(((3-(2-Aminoethyl)-1H-indol-5-yl)oxy)acetyl)-L-tyrosylglycinamid (IS159) oder ein pharmazeutisch verträgliches Derivat davon ist.

7. Verwendung gemäß Anspruch 5 oder Anspruch 6, wobei der 5HT₁-Agonist Sumatriptan oder Naratriptan oder ein pharmazeutisch verträgliches Derivat davon ist.

8. Verwendung gemäß Anspruch 1 oder Anspruch 2, wobei der Adenosin-A1-Agonist (2S, 3S, 4R, 5R)-2-(5-tert.-Butyl-[1,3,4]oxadiazol-2-yl)-5-[6-(4-chlor-2-fluorphenylamino)purin-9-yl]tetrahydrofuran-3,4-diol und der 5HT₁-Agonist Sumatriptan oder Naratriptan oder ein pharmazeutisch verträgliches Derivat davon ist.

9. Arzneimittel, welches einen Adenosin-A1-Agonisten oder ein pharmazeutisch verträgliches Derivat davon und einen 5HT₁-Agonisten oder ein pharmazeutisch verträgliches Derivat davon umfasst.

10. Arzneimittel gemäß Anspruch 9, angepasst an die orale Verabreichung.

11. Packung für Patienten umfassend einen Adenosin-A1-Agonisten oder ein pharmazeutisch verträgliches Derivat davon und einen 5HT₁-Agonisten oder ein pharmazeutisch verträgliches Derivat davon.

## Revendications

1. Utilisation d'un agoniste d'adénosine A1 ou d'un de ses dérivés pharmaceutiquement acceptables et d'un agoniste de 5HT₁ ou d'un de ses dérivés pharmaceutiquement acceptables pour la production d'un médicament destiné au traitement d'affections associés à la douleur et au soulagement des symptômes qui y sont associés.

2. Utilisation suivant la revendication 1, dans laquelle l'affection associée à la douleur est une douleur aiguë, une douleur chronique, une douleur inflammatoire, une douleur neuropathique, la douleur associée à la migraine, les céphalées provoquées par le tension, les cephalées vasculaires de Horton ou un trouble intestinal fonctionnel.

3. Utilisation suivant la revendication 1 ou la revendication 2, dans laquelle l'agoniste d'adénosine A1 est choisi entre l'adénosine, la N-(4-chloro-2-fluorophényl)-5'-O'-trifluorométhyl-adénosine, la N-[1S,trans)-2-hydroxycyclopentyl]adénosine et le (2S,3S,4R,5R)-2-(5-tertio-butyl-[1,3,4]oxadiazole-2-yl)-5-[6-(4-chloro-2-fluorophénylamino)-purine-9-yl]-tétrahydrofuranne-3,4-diol ou un de ses dérivés pharmaceutiquement acceptables.

4. Utilisation suivant la revendication 3, dans laquelle l'agoniste d'adénosine A1 est le (2S,3S,4R,5R)-2-(5-tertio-butyl-[1,3,4]oxadiazole-2-yl)-5-[6-(4-chloro-2-fluorophénylamino)-purine-9-yl]-tétrahydrofuranne-3,4-diol ou un de ses dérivés pharmaceutiquement acceptables.

5. Utilisation suivant l'une quelconque des revendications 1 à 4, dans laquelle l'agoniste de 5HT₁ est : le sumatriptan, le naratriptan, le zolmitriptan, l'élétriptan, le rizatriptan, le frovatriptan, l'almotriptan, l'aniditan, la dihydroergotamine, le donitriptan, le (1S)-1-(2-(4-(4-aminocarbonyl)phényl)-1-pipérazinyl)éthyl-3,4-dihydrométhyl-1H-2-benzopyranne-6-carboxamide (PNU-142633), la N,N-diméthyl-5-(1,2,3,6-tétrahydro-1-méthyl-4-pyridinyl)-1H-indole-3-éthanamine (ALX-0646), le 4-fluoro-N-[3-(1-méthyl-4-pipéridinyl)-1H-indole-5-yl]benzamide (LY334370) ou le N-(((3-(2-aminoéthyl)-1H-indole-5-yl)oxy)acétyl)-L-tyrosyl-glycinamide (IS159) ou un de ses dérivés pharmaceutiquement acceptables.

6. Utilisation suivant la revendication 5, dans laquelle l'agoniste de 5HT₁ est le sumatriptan, le naratriptan, le zolmitriptan, l'élétriptan, le rizatriptan, le frovatriptan, l'almotriptan, l'aniditan, la N,N-diméthyl-5-(1,2,3,6-tétrahydro-1-méthyl-4-pyridinyl)-1H-indole-3-éthanamine (ALX-0646), le 4-fluoro-N-[3-(1-méthyl-4-pipéridinyl)-1H-indole-5-yl]benzamide (LY334370) ou le N-(((3-(2-aminoéthyl)-1H-indole-5-yl)oxy)acétyl)-L-tyrosyl-glycinamide (IS159) ou un de ses dérivés pharmaceutiquement acceptables.

7. Utilisation suivant la revendication 5 ou la revendication 6, dans laquelle l'agoniste de 5HT₁ est le sumatriptan ou le naratriptan ou un de ses dérivés pharmaceutiquement acceptables.

8. Utilisation suivant la revendication 1 ou la revendication 2, dans laquelle l'agoniste d'adénosine A1 est le (2S,3S,4R,5R)-2-(5-tertio-butyl-[1,3,4]oxadiazole-2-yl)-5-[6-(4-chloro-2-fluorophénylamino)-purine-9-yl]-tétrahydrofuranne-3,4-diol et l'agoniste de 5HT est le sumatriptan ou le naratriptan ou un de ses dérivés pharmaceutiquement acceptables.

9. Composition pharmaceutique qui comprend un agoniste d'adénosine A1 ou un de ses dérivés pharmaceutiquement acceptables et un agoniste de 5HT₁ ou un de ses dérivés pharmaceutiquement acceptables.

10. Composition pharmaceutique suivant la revendication 9, adaptée à l'administration orale.

11. Conditionnement pour un patient, comprenant un agoniste d'adénosine A1 ou un de ses dérivés. pharmaceutiquement acceptables et un agoniste de 5HT₁ ou un de ses dérivés pharmaceutiquement acceptables.
